**Europäisches Patentamt**

(19) **European Patent Office**

**Office européen des brevets**

(11) Publication number: **0 233 385**
**A1**

## (12) EUROPEAN PATENT APPLICATION

(21) Application number: **86202387.6**

(22) Date of filing: **29.12.86**

(51) Int. Cl.⁴: **G01N 33/548** , **B01L 3/00**

A request for correction of some typographical errors in claims 1,8 has been filed pursuant to Rule 88 EPC. A decision on the request will be taken during the proceedings before the Examining Division (Guidelines for Examination in the EPO, A-V, 2.2).

The title of the invention has been amended (Guidelines for Examination in the EPO, A-III, 7.3).

(30) Priority: **13.01.86 NL 8600056**

(43) Date of publication of application:
**26.08.87 Bulletin 87/35**

(84) Designated Contracting States:
**AT BE CH DE ES FR GB GR IT LI LU NL SE**

(71) Applicant: **DE STAAT DER NEDERLANDEN VERTEGENWOORDIGD DOOR DE MINISTER VAN WELZIJN, VOLKSGEZONDHEID EN CULTUUR**
**P.O. Box 439**
**NL-2260 AK Leidschendam(NL)**

(72) Inventor: **Van Eijk, Ronald Victor Wilhelmus**
**Hoefijzerlaan 22**
**NL-3981 GM Bunnik(NL)**
Inventor: **Ijsselmuiden, Otto Emmamuel**
**Segherstraat 12**
**NL-2525 BV Den Haag(NL)**

(74) Representative: **van der Beek, George Frans et al**
**Nederlandsch Octrooibureau Johan de Wittlaan 15 P.O. Box 29720**
**NL-2502 LS 's-Gravenhage(NL)**

(54) **Method for detecting a member of a ligand-receptor pair, method for the preparation of a carrier to which this member is bonded and analysis equipment therefor.**

(57) A method for detecting a member of a ligand-receptor pair, such as an antigen-antibody pair, in which a carrier of porous material, to which the member of the ligand-receptor pair to be detected is bonded, is brought into contact with a liquid (to be analysed) in which the other member (to be detected) is present or is suspected to be present, and the formation of the ligand-receptor complex is detected, wherein the liquid to be analysed is made to pass at a regulated velocity through the carrier during the reaction in which the formation of the complex takes place, the preparation of such a carrier as well as an analysis equipment suitable for carrying out such a method (dot immunobinding assay).

**Method for detecting a member of a ligand-receptor pair, method for the preparation of a carrier to which a member of a ligand-receptor pair is bonded and also analysis equipment suitable for carrying out such methods.**

The invention relates to a method for detecting a member of a ligand-receptor pair in which a carrier of porous material, to which the member of the ligand-receptor pair not to be detected is bonded, is brought into contact with a liquid (to be analyzed) in which the other member (to be detected) is present or is suspected to be present, and the formation of the ligand-receptor complex is detected. A ligand-receptor pair is also in fact termed a ligand-antiligand pair and may comprise an immunological pair consisting of an antigen and an antibody.

Such a method in the context of an immunological pair, which is also termed dot immunobinding assay, is known from Hawkes et al., Analytical Biochemistry 119, 142-147 (1982). This method can be regarded as an enzyme immunoassay in which a membrane filter, for example of nitrocellulose, is used as a fixed carrier. The immobilization of an antigen or antibody (which together form an immunological pair) on the nitrocellulose proceeds rapidly and in view of the small surface area of the site to which the liquid to be analysed is applied, the quantity of antigen or antibody respectively which is needed is considerably less than that required for the enzyme immunoassay. The sensitivity of such a test system is greater or at least equal to that of the enzyme immunoassay. The use of nitrocellulose as a carrier material makes it possible, in addition, to assess the test results with respect to a background. As a result it is easier to distinguish specific and non-specific reactions from each other.

The incubation process described by Hawkes et al., proceeds as in the case of the usual enzyme immunoassays : a carrier with the immobilized antigen or antibody on it is brought into contact with a solution in which antibodies or antigens respectively are present. The antibodies bonded to the antigen or the antigens bound to the antibody are then detected by means of a second antibody with an enzyme coupled to it and a colour reaction. The antibodies have to come into contact with the (immobilized) antigen by diffusion. For this process it is necessary for incubation to be carried out for at least 1 hour.

It has now been found that the incubation time can be considerably shortened if, in a method as described in the introduction, the liquid to be analysed is made to pass at a regulated velocity through the carrier during the reaction in which the formation of the complex takes place. Said regulated velocity can be adjusted, according to the invention, by means of an overpressure or an underpressure. A member of the ligand-receptor pair may be bonded in this process in a point form, in a line-tyoe or in another pattern to the carrier.

In the method according to the invention an antigen or antibody bonding reaction, for example, takes place with continuous pressure or suction filtration on a carrier made of porous material. Such a carrier may, for example, be a membrane filter made of nitrocellulose, cellulose (acetate), polyamide and the like. The reaction time, which is 10-15 times shorter than the incubation time according to the method of the prior art, is possible because the substances to be reacted are moved forcibly towards each other, which proceeds much more rapidly than if said substances have to move towards each other by diffusion, as occurs in a normal enzyme immunoassay.

The velocity with which the liquid to be analysed is made to pass, according to the invention, through the carrier, is preferably adjusted in a manner such that a detectable quantity of complex is formed. In general, in the case of an antibody-antigen reaction, this velocity will be 0.1-50 ml/cm²/min.

Preferably, in the last named case, polyoxyethylene sorbitan monolaurate is added to the sample to be analysed to prevent non-specific bonding of antibodies or antigens respectively to the carrier material. It has been found that, for example, a polyoxyethylene sorbitan monolaurate marketed under the description Tween 20 in this case gives better results than for example bovine serum albumin. Tween 20 is added preferably in a concentration between 0.05 and 1 % by volume.

The invention also relates to a method for the preparation of a carrier to which antigen is bonded, and which carrier can be used in the method described above. Said method is characterized in that, in the case of noncovalent bonding, the liquid which contains the antigen is applied to the carrier and is immediately sucked through the carrier (by application of underpressure) and in that, in the case of covalent bonding, the liquid which contains the antigen or antibody is applied to an activated carrier, for example a nitrocellulose carrier treated with glutardialdehyde or another bifunctional reagent.

In the last named method a detergent is preferably added in a quantity below the critical micelle concentration (CMC), as a result of which an improvement of the complex formation of the immunological pair can be brought about. For this purpose a detergent marketed under the description Zwittergent 3-14 (3-tetradecyldimethylammonio-1-propane sulphate), octyl-$\beta$-D-glycopyranoside, octyl-$\beta$-D-thioglycocyranoside etc, for example, are used as detergent.

The method for detecting antigens or antibodies according to the enzyme immunofiltration method according to the invention is carried out for a non-competitive assay by means of the following steps:

a) moistening of a dry carrier, such as a nitrocellulose membrane,

b) either application of a liquid containing an antigen or antibody respectively, which liquid is immediately sucked through the carrier, the antigen-containing liquid optionally being allowed to contain a quantity of detergent below the CMC, or covalent bonding of the antigen or the antibody to an activated carrier, such as a nitrocellulose carrier pretreated with 0.1 % by weight of glutardialdehyde,

c) optional storage of the carrier at low temperature, for example 4°C, in a sealed container,

d) optional moistening of the carrier with a solution containing, for example, 0.5 % by volume of Tween 20 underpressure in order to occupy sites still open on the carrier and thereby to prevent non-specific bonding to the carrier,

e) application of a sample, i.e. a liquid containing a first antibody or antigen respectively or a liquid in which a first antibody or antigen respectively is suspected to be present, to the carrier containing antigen or antibody respectively, the sample being sucked by means of under-or overpressure at a regulated velocity of 0.1 -50 ml/cm²/min through the carrier, Tween 20 preferably being added to the sample in a concentration between 0.05 and 1 % by volume,

f) washing of the carrier with water to which 0.5 % by volume of Tween 20 has optionally been added, the wash liquid being sucked or pressed through the carrier,

g) addition of an antibody labelled with a signal-producing system and sucking and/or pressing of said liquid through the carrier, as described under step e), it being possible for step g) to be preceded optionally by addition of a non-labelled antibody, as described under step e), Tween 20 preferably being added to the sample in a concentration between 0.05 and 1 % by volume,

h) washing of the carrier as under step f),

i) drying of the carrier by suction, and determination of the quantity of labelled complex on the carrier according to a method of measurement which is adapted to the signal producing system which has been used. In this connection, for example spectrophotometry, radioactivity measurement, microscopy or a (computer-controlled) television camera can be used.

In the step a) described above Zwittergent 3-14 is preferably used.

In the step g) described above the signal-producing system may be, for example, one of the following categories: chromogens, catalysed reactions, radioactive labels, etc.

In the case of a competitive assay, the steps a) -f) described above are first carried out and then the step g):

g) application of an antibody or antigen respectively labelled with a signal-producing system to the carrier, the sample being sucked or pressed by means of under-or over-pressure through the carrier at a regulated velocity between 0.1 and 50 ml/cm²/min, Tween 20 preferably being added to the sample in a concentration between 0.05 and 1 % by volume, after which the steps h) and i) described above of the non-competitive assay are carried out.

The invention also relates to analysis equipment comprising in the operational state, viewed from top to bottom, a sample-receiving plate provided with a number of holes, a carrier material, seals (gaskets) being fitted between the carrier material and the sample-receiving plate at the position of the holes, and a support plate provided with holes which, in the fitted state, are in line with the holes of the sample-receiving plate, the support plate being provided with a connection for placing it under reduced pressure, which equipment is characterized in that, between the layer of carrier material and the support plate at the position of the holes in the support plate, further seals are fitted which are in line with the abovementioned seals and essentially of the same average inside diameter. In the support plate grooves are preferably provided for the partial reception of further seals. The seals preferably comprise 0-rings, but oval or oblong rings of deformable material can also be used. Instead of the latter, it is also possible to fit two layers of readily deformable material, for example polysiloxane rubber in which holes have been punched, in the equipment, between which layers the carrier material is clamped. The holes in such deformable layers should of course be in line with the holes in the sample-receiving plate and the suoport plate.

Oblong seals may be used with advantage in the case of the so-called immunoblot technique, in which oblong reaction spots are present.

In the method according to the invention the reaction area, in which the formation of the ligand-receptor pair takes place, should preferably be surrounded by an edge of the carrier (filter) which is clamped between two narrow strips of deformable material in a manner such that the pores of the carrier are completely closed by pressure in order to prevent lateral capillary flow. It will be clear that, at the level of the edge, a fairly high pressure has to be exerted and that therefore deformable material is preferably used on both sides of the edge in order to prevent capillary action.

An optimum seal is obtained if an elevation is provided at the position of the seals. By this is meant that, over a relatively narrow area around the holes, thick sections are made, either on the seal itself, for example a "rimple" on an oblong packing, or on the sample-receiving and/or support plate.

Using the method or the equipment according to the present invention, it is possible to perform immunological analyses very rapidly. This is of importance, for example, in the case of sexually transmittable diseases (STD). Not only the number of patients who suffer from a STD, but also the number of different infectious agents has increased considerably in recent years. These agents are to be found among the bacteria, protozoa, fungi and viruses. A wide range of different syndromes is provoked by said infectious agents, it being striking that different agents manifest themselves clinically more or less in the same way. The result is therefore that a correct diagnosis by clinical investigation alone is not possible and that laboratory investigation is the most important support factor for the latter. By means of the method(s) and the equipment according to the invention, it is now possible to perform such an immunological laboratory investigation so rapidly that the patient concerned can wait for the result and a new appointment does not have to be made for possibly continuing the therapy. It is pointed out in this connection that the usual enzyme immunoassay (ELISA) requires at least 3 hours before the test result becomes known. The test result of the method according to the invention can be obtained within 5-20 minutes.

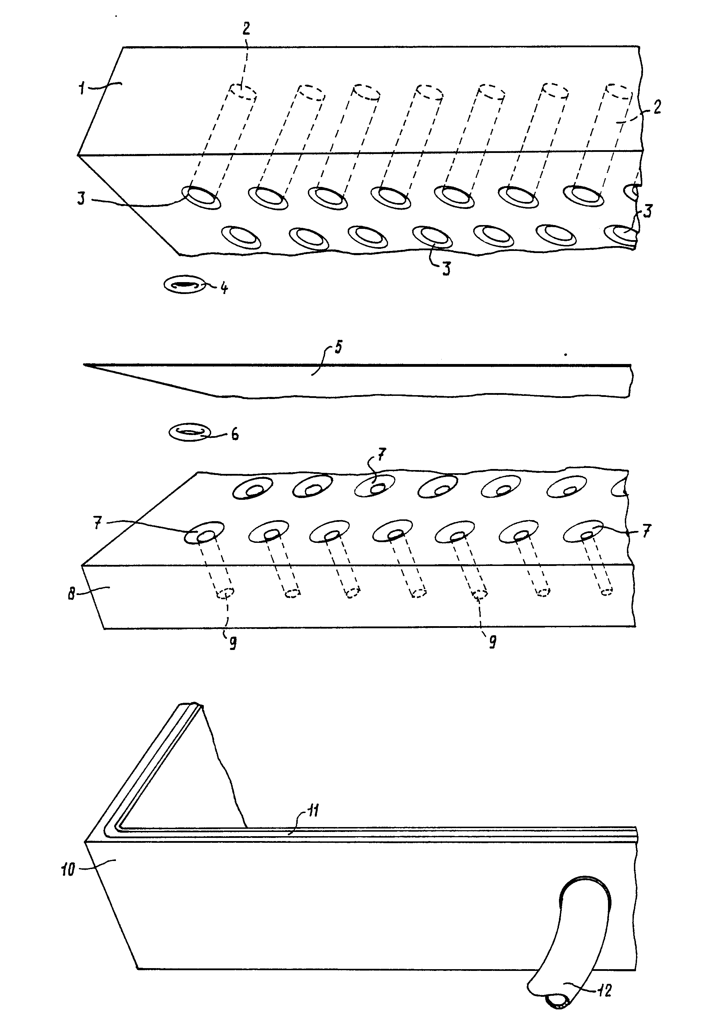

The analysis equipment according to the invention will now be described in more detail on the basis of an exemplary embodiment shown in the drawing. In the drawing an analysis equipment is shown in cut-away form. It consists of a sample-receiving plate 1 provided with holes 2 in which the liquid is applied. Said holes 2 are provided from below with an annular groove 3 in which 0-rings 4 may partially lie. In the assembled state the 0-rings 4 are adjoined by a carrier material 5 which is in turn adjoined by 0-rings 6 which lie partially in grooves 7 provided in a support plate 8. In said support plate 8 there are provided holes 9 which lie in line with the annular grooves 7. Said support plate 8 may be fitted on a container 10, suitable sealing means 11 being present in order to ensure sealing between support plate 8 and container 10. The pipe 12 emerging from said container 10 can be connected to a vacuum pump means.

The analysis equipment according to the invention can be made by adapting an equipment obtainable commercially for performing dot immunobonding assays, for example microsample filtering manifold made by Schleicher & Schuell, West Germany.

The analysis equipment described above is used as follows: small spots of antigen or antibody suspension are applied under high vacuum to a piece of nitrocellulose used as carrier material. In doing this, use may be made of a template. The carrier material is then clamped between the support plate and the sample-receiving plate. The first antibody or antigen can then be made to incubate by rapidly introducing the antibody solution into the small incubation cups, formed by the holes 2, by means of a multichannel pipette. At the same time, the air inlet should be opened in order to bring the pressure in the container 10 to equilibrium with the atmospheric pressure. After pipetting, the air inlet is closed and the vacuum pump connected to pipe 12 is started up. The latter is preferably a calibrate peristaltic vacuum pump and at the same time, the container 10 is largely filled with liquid so that precise operation of the suction filtration is obtained through the intake of liquid by the vacuum pump. The antigen-antibooy reaction will not take place by means of diffusion, as in a normal ELISA, but by continuous suction filtration at the level of the nitrocellulose membrane used as carrier material 5. Tnis makes very short reaction times possible. In this connection it is surprising that, as a result of the fitting of an 0-ring between the support plate and the layer of carrier material, no leakage takes place. For the conjugate and substrate step the procedure is similar to a normal ELISA.

It is also possible to collect the filtrate separately if a half microtitration plate is placed in the vacuum chamber with a pipe system for liquid in addition. The removal of the liquid can best take place via an air/liquid separation flask to which a quantity of chlorbleachinglye has been added as disinfectant.

Although the embodiment described above is a preferred embodiment of the invention, it must be understood that the invention is not limited thereto and that those skilled in the art can make changes thereto without falling outside the concept of the present invention.

In the following examples the invention is explained in more detail.

Example I

Antibody detection (non-competitive )

In performing the analysis use is made of the analysis equipment described above.

A nitrocellulose membrane (pore size 0.45 μm) is moistened with distilled water. To each antigen spot, 5 μm of a suspension of Treponema pallidum organisms (20 μg/ml of protein) in phosphate buffered saline (PBS) to which 0.005 % w/v of Zwittergent 3-14 has been added is applied under vacuum. The carrier can be stored at 4°C in a sealed box or can be used after drying for approximately 1 minute. Each spot is washed under vacuum with 200 μl of PBS to which 0.5 % v/v of Tween 20 (PBS-Tween) has been added. 200 μl of patient serum diluted 1:50 in PBS-Tween is then applied, the serum sample being sucked through the carrier by means of underpressure at a regulated velocity of 0.2 ml/cm².min. Washing is then carried out with 200 μl of PBS-Tween, after which 200 μl of goat-antihuman immunoglobulin with horse-radish peroxidase and diluted 1:20,000 in PBS-Tween is sucked through the carrier at a regulated velocity of 0.2 ml/cm²/min. The carrier is washed three times under vacuum with 200 μl of PBS-Tween. 200 μl of substrate solution, which contains tetramethylbenzidine (1.2 mg/ml), hydrogen peroxide (0.003 %), sodium acetate - (6.8%) and citric acid (1.4 %), is then applied and sucked through after incubation for 3 minutes. The appearance of a greenish blue spot against a white background indicates a positive reaction. The intensity of the colour reaction can be indicated in gradations of 1 + and 2 +, the 1 + reaction being the slightest degree of colouration which can still be regarded as positive. Results of serum samples originating from syphilitic and non syphilitic patients are shown in Table A.

TABLE A

| diagnosis | number of serum samples | number (%) of positive serum samples |
|---|---|---|
| syphilis | 151 | 146 (96.7) |
| no syphilis | 504 | 3 (0.6) |

Example II

Antibody detection (competitive)

The performance of the analysis proceeds similarly to that of the non-competitive antibody detection described above. After reaction of the patient serum and the washing step with PBS-Tween which follows, 200 μl of monoclonal antibody conjugated with horse-radish peroxidase, directed against Treponema pallidum and diluted 1:20,000 in PBS-Tween is applied and sucked through the carrier at a regulated velocity of 0.2 ml/cm²/min. After washing three times with 200 μl of PBS-Tween, 200 μl of substrate solution is applied as described in the case of non-competitive antibody detection, and sucked through after incubation for 3 minutes. The intensity of the colour reaction is compared with an antigen spot which has been incubated with the monoclonal conjugated with horse-radish peroxidase. In the event of inhibition by antibodies in the patient serum, the intensity of the colour reaction changes from 2 + to 1 + or to negative. The results obtained with some serum samples from syphilitic and non-syphilitic patients are shown in Table B.

## TABLE B

| diagnosis | number of serum samples | number of serum samples (%) which exhibit inhibition |
|---|---|---|
| syphilis | 10 | 8 (80) |
| no syphilis | 10 | 0 (0) |

Example III

Antigen detection (non-competitive)

For each spot, 5 $\mu$l of monoclonal antibody directed against Treponema pallidum diluted 1:200 in PBS is applied under vacuum. After drying for approximately 1 minute, washing is carried out under vacuum with 200 $\mu$l of PBS-Tween. 200 $\mu$l of a suspension of Treponema pallidum organisms in PBS, to which 0.05 % Tween 20 has been added, is then applied and sucked through the carrier at a regulated velocity of 0.2 ml/cm²/min. A suspension of E. coli organisms is used as control. The carrier is washed under vacuum with 200 $\mu$l of PBS-Tween. 200 $\mu$l of monochloral antibody, conjugated with horse-radish peroxidase, directed against Treponema pallidum diluted 1:10,000 in PBS-Tween is then applied and sucked through the carrier at a regulated velocity of 0.2 ml/cm²/min. After washing three times under vacuum with 200 $\mu$l of PBS-Tween 200 $\mu$l of substrate solution as described in Example I is added and sucked through after incubation for 3 minutes. The appearance of a greenish blue soot against a white background indicates the presence of Treponema pallidum in the suspension under investigation. The results with various quantities of Treponema pallidum organisms are shown in Table C. The controls with E. coli revealed a negative result.

## TABLE C

| number of Treponema pallidum organisms per ml | test result |
|---|---|
| $1 \times 10^8$ | pos. |
| $5 \times 10^7$ | pos. |
| $1 \times 10^7$ | pos. |
| $5 \times 10^6$ | neg. |
| $1 \times 10^6$ | neg. |

## Claims

1. Method for detecting a member of a ligand-receptor pair in which a carrier of porous material, to which the member of the ligand-receptor pair to be detected is bonded, is brought into contact with a liquid (to be analysed) in which the other member (to be detected) is present or is suspected to be present, and the formation of the ligand-receptor complex is detected, characterized in that the liquid to be analysed is made to pass at a regulated velocity through the carrier during the reaction in which the formation of the complex takes place.

2. Method according to Claim 1, characterized in that the velocity at which the liquid to be analysed is made to pass through the carrier is adjusted in a manner such that at least a detectable quantity of complex is formed.

3. Method according to Claim 1 or 2, the ligand-receptor pair being an antigen-antibody pair, characterized in that the velocity at which the liquid to be analysed is made to pass through the carrier is adjusted between 0.1 and 50 ml/cm²/min.

4. Method according to Claim 2 or 3, characterized in that polyoxyethylene sorbitan monolaurate is added to the sample to be analysed in a concentration between 0.05 and 1 % by volume.

5. Method for the preparation of a carrier, to which a member of a ligand-receptor pair has been covalently or non-covalently bonded, for use in the method according to one or more of Claims 1 -4, characterized in that, in the case of covalent bonding, the liquid which contains said member is applied to an activated carrier, and in that, in the case of non-covalent bonding, the liquid which contains said member is applied to the non-activated carrier and immediately sucked through the carrier.

6. Method according to Claim 5, characterized in that an improvement of the complex formation of the ligand-receptor pair is brought about by adding a detergent in a quantity below the critical micelle concentration (CMC).

7. Method for detecting antigens or antibodies according to the enzyme immunofiltration method, characterized in that the following steps are performed for a non-competitive assay:

a) moistening of a dry carrier, such as a nitrocellulose membrane,

b) either application of a liquid containing an antigen or antibody respectively, which liquid is immediately sucked through the carrier, the antigen-containing liquid optionally being allowed to contain a quantity of detergent below the CMC, or covalent bonding of the antigen or the antibody to an activated carrier, such as an nitrocellulose carrier pretreated with glutardialdehyde,

c) optional storage of the carrier at 4°C in a sealed container,

d) optional moistening of the carrier with a solution containing Tween 20 under under-pressure in order to occupy sites still open on the carrier and thereby to prevent non-specific bonding to the carrier,

e) application of a sample, i.e. a liquid containing a first antibody or antigen respectively or a liquid in which a first antibody or antigen respectively is suspected to be present, to the carrier containing antigen or antibody respectively, the sample being sucked or pressed by means of under-or overpressure at a regulated velocity of 0.1 -50 ml/cm²/min. through the carrier, Tween 20 preferably being added to the sample in a concentration between 0.05 and 1 % by volume,

f) washing of the carrier with water to which 0.5% by volume of Tween 20 has optionally been added, the wash liquid being sucked or pressed through the carrier,

g) addition of an antibody labelled with a signal-producing system and sucking or pressing of said liquid through the carrier as described under step e), it being possible for step g) to be preceded optionally by addition of a non-labelled antibody as described under step e), Tween 20 preferably being added to the sample in a concentration between 0.05 and 1 % by volume,

h) washing of the carrier as under step f),

i) drying of the carrier by suction, and determination of the quantity of labelled complex on the carrier according to a method of measurement which is adapted to the signal-producing system which has been used.

8. Method for detecting antigen or antibodies according to the enzyme immunofiltration method characterized in that the following steps are performed for a non-competitive assay:

a) analogous to step a) of Claim 7,

b) analogous to step b) of Claim 7,

c) analogous to step c) of Claim 7,

d) analogous to step d) of Claim 7,

e) analogous to step e) of Claim 7,

f) analogous to step f) of Claim 7,

g) application of an antibody or antigen respectively labelled with a signal-producing system, the sample being sucked or pressed by means of under-or over-pressure through the carrier at a regulated velocity between 0.1 and 50 ml/cm².min, Tween 20 preferably being added to the sample in a concentration between 0.05 and 1 % by volume,

h) analogous to step f) of Claim 7,

i) analogous to step i) of Claim 7,

9. Analysis equipment comprising in the operational state, viewed from top to bottom, a sample-receiving plate provided with a number of holes, a carrier material, seals being fitted between the carrier material and the sample-receiving plate at the position of the holes, and a support plate provided with holes which, in the fitted state, are in line with the holes of the sample-receiving plate, the support plate being provided with a connection for placing it under reduced pressure, characterized in that between the layer of carrier material and the support plate at the position of the holes in the support plate, further seals are fitted which are in line with the abovementioned seals and essentially have the same average inside diameter.

10. Analysis equipment according to Claim 9, characterized in that grooves are provided in the support plate for partially receiving further seals.

11. Analysis equioment according to Claim 9 or 10, characterized in that the seals comprise 0-rings, oval rings or oblong rings of deformable material.

12. Analysis equipment according to Claims 9-11, characterized in that the seals comprise at least two layers of deformable material.

13. Analysis equipment according to Claims 9-12, characterized in that an elevation is provided at the position of the sealing surface of the seals.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | DE-A-3 346 795 (W. KINZEBACH) <br> * Claims 1-8; pages 11-13, example * | 1,7,8 | G 01 N 33/548 <br> B 01 L 3/00 |
| Y | US-A-4 427 415 (P.H. CLEVELAND) <br> * Figures: page 1, column 2, lines 17-40; page 2, column 3, lines 1-30; claims * | 1,7,8 | |
| Y | US-A-4 031 197 (V.A. MARINKOVICH) <br> * Figures 6,7,9; pages 2-4; claim 6 * | 1,7,8 | |
| X | | 1,2,9 | |
| A | EP-A-0 133 272 (BOEHRINGER MANNHEIM) <br> * Page 3 * | 4,7 | TECHNICAL FIELDS SEARCHED (Int. Cl.4) <br><br> G 01 N <br> C 12 G <br> B 01 L |
| A | EP-A-0 118 735 (MILLIPORE CORP.) <br> * Whole document * | 9-13 | |
| A | EP-A-0 119 858 (MAST IMMUNOSYSTEMS LTD) <br> * Claims; figures * | 9-13 | |

-/-

The present search report has been drawn up for all claims

| Place of search | Date of completion of the search | Examiner |
|---|---|---|
| THE HAGUE | 15-04-1987 | MEYLAERTS H. |

## DOCUMENTS CONSIDERED TO BE RELEVANT

Page 2

| Category | Citation of document with indication, where appropriate, of relevant passages | Relevant to claim | CLASSIFICATION OF THE APPLICATION (Int. Cl.4) |
|---|---|---|---|
| A | WO-A-8 505 451 (HIBRITECH INC.) <br> * Pages 7-9; figure 1 * <br><br> ----- | 1,9 | |
| | | | **TECHNICAL FIELDS SEARCHED (Int. Cl.4)** |

The present search report has been drawn up for all claims

| Place of search | Date of completion of the search | Examiner |
|---|---|---|
| THE HAGUE | 15-04-1987 | MEYLAERTS H. |